# EUROPEAN PATENT APPLICATION

(11) **EP 4 725 603 A1**
(43) Date of publication of application: **15.04.2026**
(21) Application number: 25207719.3
(22) Date of filing: 09.10.2025
(51) Int. Cl.: B01L 3/00, C12M 3/00

(54) **DEVICE FOR CONTROLLED PLACEMENT, CULTURE, EXPLOSURE AND PERMEABILITY EVALUATION OF EX VIVO EPITHELIAL TISSUE**

(30) Priority: 09.10.2024 GR 20240100710
(71) Applicant: Aristotle University of Thessaloniki - E.L.K.E. (Eidikos Logariasmos Kondilion Erevnas), 54636 Thessaloniki (GR)
(72) Inventor: BAKOPOULOU, Athina, 54624 Thessaloniki (GR); MACHLA, Fotini, 54621 Thessaloniki (GR); FATOUROS, Dimitrios, 55121 Thessaloniki (GR)
(74) Representative: Petsis, Christos

(57) **Abstract**

The invention is a device for the controlled placement, cultivation, exposure and permeability evaluation of *ex vivo* epithelial tissues for diagnostic or research purposes. The device consists of an upper (Figures 4-6) and a lower (Figures 1-4) part, which are assembled with the tissue and with each other. The resulting arrangement has a triple utility in one device. Firstly, tissue insertion and culture can be done in an oriented and controlled manner at an air-liquid interface condition. Secondly, the device accommodates spatially controlled exposure of the tissue to exogenous factors that come into contact only with the epithelial side of the tissue and allows the assessment of the under evaluation substances' effect towards the tissue. Finally, without changing the setup, tissue permeability can be tested. The device allows the evaluation of small epithelial tissue sections, less than 5 millimeters in diameter, and they can be placed in 24-well culture plates.

## Description

### Field of the invention

The invention (referred herein as Epi-ExPer device) relates to a device for controlled placement, culturing, exposure to exogenous factors, and permeability evaluation of *ex vivo* epithelial tissues for diagnostic or research purposes.

Specifically, the Epi-ExPer device can be utilized by research centers in the industrial sector and especially by the Research and Development (R&D) departments of the Pharmaceutical Industry. Companies developing new pharmaceutical formulations with a transdermal or transmucosal route of administration can utilize the Epi-ExPer device for easier and faster exposure and assessment of epithelial tissue permeability.

Pharmaceutical agents under development must be evaluated for their effect on the permeability and viability of targeted tissues. In this area, the multiple role of the Epi-ExPer device and the potential to increase the ratio of research results : resources (high-throughput models) is particularly important.

In academia, the Epi-ExPer device can be utilized for research purposes. In addition to being used to answer research questions in primarily *ex vivo* studies, the device can be additionally used as a tool to assess tissue permeability in clinical studies, and to assist data collection that will lead to the correlation of pathological conditions with tissue permeability indices (e.g. permeability of healthy versus pathological tissues).

Similarly, the Epi-ExPer device can be utilized in the field of diagnostics. Diagnostic and clinical laboratories can utilize the device to diagnose pathological diseases, after taking a biopsy from the patient.

### Prior Art

The state of the art includes already known *ex vivo* organ culture, exposure and epithelial barrier permeability evaluation systems, which are mentioned below. The *ex vivo* organ culture and subsequently the epithelial tissue culture in culture dishes can so far be performed using the following methods: using the plasma clot (plasma clot or watch glass) method, using the agar gel method, using the raft method, and using the grid method. [1].

While the above techniques allow the cultivation of organs/tissues, either in full contact with a nutrient medium or at an air-liquid interface, they do not facilitate the spatially controlled exposure of tissues to exogenous factors. *Ex vivo* tissue exposure should mimic the natural *in vivo* tissue exposure. For example, exposure of transdermal agents should only occur in contact with the outer surface of the epithelium and not in contact with the underlying dermis. The effect of various exogenous stimuli is a subject of studies in the field of synthesis of new biomaterials and pharmaceutical agents. For example, pharmaceutical formulations with transdermal/transmucosal route of administration are directly affected by the mechanism and rate of diffusion of pharmaceutical substances through the targeted epithelial barrier.

In the above culture techniques, exposure to exogenous stimuli in liquid form can be performed using the 'drop' exposure technique of the exogenous factor under investigation. That is, the liquid exogenous agent in question (e.g. toxic monomers of medical biomaterials, pharmaceutical transdermal/transmucosal formulations under development) can be placed in a small amount (50 - 100 µl) on the outer surface of the epithelial tissue.

This poses the risk of the drop moving during the transfer of the culture plate to and from the culture incubator. At the same time, existing models do not ensure spatial exposure of exogenous factors, and the droplet exposure method does not provide a uniform distribution of the factor on the epithelial surface, as at its periphery, the drop gathers a smaller amount of liquid.

For this purpose, cylinders can be used as an alternative exposure system [2] e.g. silicone ('cloning rings') that are placed on the epithelial tissue with the help of an insulating 'glue' material, such as silicone glue/vacuum grease/high-vacuum silicon grease [3]. These rings are intended for other laboratory procedures, mainly the isolation of colonies ('clones', hence the name of the rings) [4], but they can also be used for the exposure of exogenous factors to epithelial tissue.

The 'cloning ring', which is an additional accessory, carries the risk of the ring moving around on the tissue and, above all, the risk of the glue remaining on the tissue section during the exposure and evaluation stages of the results. Removal of the remaining adhesive from the epithelial tissue after the exposure period may lead to epithelial injury and a false increase of its permeability. This problem has been reported in the literature and a solution in cases of isolation of cell colonies is the application of an agarose gel around the ring [5]. The duration of use of the ring in cases of colony isolation is 5 minutes and the use of agarose actually replaces the use of the 'glue' from the plate and at the same time spatially limits the exposure of the cell detachment factor. However, in the case of exposure of epithelial tissues to exogenous factors, agarose is not a possible solution, as agarose is permeable to exogenous factors and the duration of exposure to exogenous factors lasts several hours, depending on the experimental protocol.

For testing the permeability of epithelial tissues, the most widely used tool is the Franz vertical diffusion cell/chamber [6]. The Franz cell is used for tissue sections larger than 6-7 mm in diameter, as the smallest cell has a diffusion hole of 5 mm in diameter [7]. Models aiming at modifying/improving Franz cells have been found in the literature, but they differ from the purposes of the Epi-ExPer device [8-11]. These variations, while improving some of the characteristics of the standard Franz cell, do not significantly modify their mode of operation.

While Franz cells are useful for testing the permeability of animal epithelial tissue sections where the tissues can be dissected and cut to the desired dimensions, they are difficult to use in cases of human tissue sections. Taking large tissue samples from humans is unnecessary, causes unnecessary harm and discomfort to the donor, and conflicts with ethical and moral principles of research. Furthermore, in the above existing techniques, after the completion of exposure of the factors under investigation to the tissue sections, in order to check for changes in the permeability of the epithelial barrier, it is necessary to transfer the tissue sections to another arrangement, where the sections will be repositioned to investigate their permeability.

### Aim of the Invention

The Epi-ExPer device was designed to address the aforementioned disadvantages of existing *ex vivo* epithelial tissue culture, exposure and permeability control techniques.

The disadvantages of existing techniques for exposing epithelial tissues to exogenous factors (risk of droplet movement and risk of ring adhesive remaining) are circumvented with the Epi-ExPer device, as the Epi-ExPer device has a designed well for exposure to exogenous factors, which lies on the outer surface of the tissue section via an elastic ring.

Also, the disadvantage of disassembling the culture and exposure device, and reassembling the tissue section in the permeability control device, is circumvented by the present Epi-ExPer device. The Epi-ExPer device was designed to provide tissue placement during culture and exposure, and in the same structure, without disassembling the device or removing the tissue, it is possible to investigate the effect of external factors on the permeability of the piece within the culture plate.

### Summary of the Invention

Thanks to the invention, there is provided a Device for the controlled placement, cultivation, exposure and permeability assessment of *ex vivo* epithelial tissues, that comprises an upper part and a lower part wherein on the one hand the upper part includes a retaining cylinder which fits on the periphery of the upper surface of the epithelial tissue piece and holds it in contact with the lower part, particularly wherein on this upper part a flat cylinder is placed for the support of three cantilevers that hold the upper edge of the rubber bands, and where further the above tissue holding cylinder extends upwards in such a way as to create a well, which either remains empty in the case of tissue culture in contact with air, or receives the chemical exogenous exposure factors under study, and on the other hand, the lower part includes a cylinder which constitutes the support plane of the tissue and which has a hole in its center from which the tissue is supplied with nutrients, as it comes into contact with the underlying nutrient medium through it, where in particular a hole is provided above it, and further above it a second cylindrical plane is provided with a bearing hole for receiving and stabilizing the above upper part of the device, where on the cylindrical plane three cantilevers are specifically provided for holding rubber bands.

According to a particular embodiment of the device of the invention, in the upper part, the holding cylinder has an outer diameter of 4 mm, an inner diameter of 3 mm and a height of 2 mm, the cylindrical plane has an outer diameter of 10 mm, a height of 1 mm, and bears in its center a hole of diameter 3 mm, which plane serves to support the above cantilevers which preferably have a diameter of 1 mm and a height of 3 mm, and where the receiving well has an inner diameter of 3 mm, an outer diameter of 4 mm and a height of 3 mm.

According to a more particular embodiment of the device of the invention, in the lower part, the cylindrical supporting plane has an outer diameter of 10 mm, an inner diameter of 3 mm and a height of 1 mm, where in particular the hole of is provided in the center preferably with a diameter of 3 mm, the second cylindrical plane has an external diameter of 10 mm, while the hole of the cylinder has a diameter of 5 mm.

According to a still more particular embodiment of the device of the invention, an additional thin ring is provided in the above upper part which is welded to the lower edge of the above tissue holding cylinder, and fits to the periphery of the upper surface of the tissue piece.

According to a yet particular embodiment of the device of the invention, an additional thin ring is provided in the lower part, which is connected to the cylindrical plane by means of three beams of preferably 0.5 mm diameter, and which ring is intended to fix the device on the edge of a 24-well culture plate.

According to an even more particular embodiment of the device of the invention, the above cylinders of the lower part are connected to each other by three beams particularly preferably with a diameter of 1 mm and a height of 2 mm.

According to a further particular embodiment of the device of the invention, the above elastic ring that is welded to the rim of the cylinder of the upper part has a height of 0.5 mm, an internal diameter of 3 mm and an external diameter of 3.5 mm.

According to another embodiment of the device of the invention, it consists of an upper and a lower part, and it is characterized by its multiple role for controlled placement, culture, exposure and permeability assessment of *ex vivo* epithelial tissues, without the need to change the device configuration between roles.

According to a specific embodiment of the invention, it is characterized by the possibility of placing, cultivating, exposing, and assessing the permeability of small epithelial tissue pieces, with a diameter even smaller than 5 mm, as the hole diameter of the plane on which the tissue is held is 3 mm.

According to a more specific embodiment of the invention, it is characterized by the possibility of culturing tissue sections in contact with a liquid-air interface, i.e. the upper part of the tissue can be cultured in contact with air through the well provided in the upper part of the device, if desired, while the lower part of the tissue piece comes into contact with the liquid nutrient medium through the hole of the plane of the lower part of the device on which the tissue sits.

According to a still more specific embodiment of the invention, it is characterized by the possibility of spatially controlled exposure of the outer surface of the epithelial tissue to exogenous factors, through the well provided in the upper part of the device. The effect of these factors on the vitality or permeability of the epithelial barrier is of research or diagnostic interest.

According to a further embodiment of the invention, it has the possibility of repeated use, as the upper and lower parts of the device can be disassembled and reassembled with a new tissue piece using thin rubber bands held on cantilevers in the upper and lower parts of the device.

According to a still more specific embodiment of the device of the invention, an additional cylindrical solid plane, 12 mm in diameter and 1 mm thick, is provided at the bottom of the device for its seating at the bottom of a well of a commercially available culture plate. In this variation, the device is placed within the culture well, rather than suspended therein via the ring by which the device sits on the rim of commercially available 24-well culture plates. The main mounting plane is fixed above the cylindrical bearing plane with three beams, each of which has a diameter of 1 mm and a height of 5 mm.

The invention has the following advantages. Unlike Franz cells, the Epi-ExPer device is designed to hold small tissue pieces, less than 5 mm in diameter. Obtaining as small pieces as possible is essential in the case of obtaining tissues from humans, but also desirable in the case of obtaining tissues from other animals. The use of laboratory animals following recent European Union directives must be subject to the 3R rule (Replacement, Reduction, Refinement) [12].

Furthermore, compared to the vertical Franz diffusion cell and its variants, the Epi-ExPer device allows the permeability assessment of barriers within standard 24-well culture plates. The vertical Franz cell, which has a diffusion hole diameter of approximately 0.9 cm, has a total device length of approximately 15 cm, a height of approximately 10 cm, and a diffusion substance collection space of approximately 5 mL volume. The dimensions make the cultivation of many tissue sections/repeats difficult. In contrast, the Epi-ExPer device with a 0.3 cm diameter diffusion hole has a total device height of less than 1.5 cm, a total diameter of approximately 1.5 cm, and a diffusion substance collection space of 2.4 mL.

The Franz cell is expensive and made of glass, therefore fragile, unlike the Epi-ExPer device. The Epi-ExPer device is easily cleaned and sterilized in bulk and does not require other components for its proper operation, such as a special base for supporting the Franz cells.

Furthermore, the small size and weight of the Epi-ExPer device allows it's easy transportation and handling, as well as an increase in the laboratory data : resource ratio (high-throughput model). In a 24-well culture plate, with overall external dimensions of 8.5 x 12.2 cm., 24 tissue sections can be cultured, exposed to exogenous factors, and tested for permeability.

It is reusable, after sterilization, compared to cell culture inserts that are disposable. In the well space under the Epi-ExPer device, an empty space has been left for the placement of a stirring magnet, which is not necessary though for its proper operation, as the receptor lower side collection volume is 2.4 mL, mixing the liquid prior to sampling is easy using a pipette (up-down pipetting). Also, the low manufacturing cost, easy packaging and distribution of the device, and durability are positive features.

The Epi-ExPer device, in relation to existing techniques, provides multiple utility in one device (controlled placement - culture - exposure - permeability control). In summary, the epithelial tissue piece is fitted once on the Epi-ExPer device and after its cultivation, no additional component, e.g. a ring, is necessary for its exposure, nor should it be transferred and attached to another device, e.g. a Franz cell, to test it's permeability.

At the same time, it allows the controlled and limited exposure of exogenous agents on the outer surface of the epithelial tissue, with uniform exposure of the agents to the desired surface, without the risk of movement of the exogenous factors under investigation, compared to the drop exposure method, and the risk of adhesive remaining on the tissue, compared to the application of silicone vacuum adhesive.

To summarize, the invention is a device for the controlled placement, cultivation, exposure and permeability evaluation of *ex vivo* epithelial tissues for diagnostic or research purposes. The device consists of an upper and a lower part, which are assembled with the tissue and with each other. The resulting arrangement has a triple utility in one device. Firstly, tissue insertion and culture can be done in an oriented and controlled manner at an air-liquid interface condition. Secondly, the device accommodates spatially controlled exposure of the tissue to exogenous factors that come into contact only with the epithelial side of the tissue and allows the assessment of the under evaluation substances' effect towards the tissue. Finally, without changing the setup, tissue permeability can be tested. The device allows the evaluation of small epithelial tissue sections, less than 5 millimeters in diameter, and they can be placed in 24-well culture plates.

### Brief description of the drawings

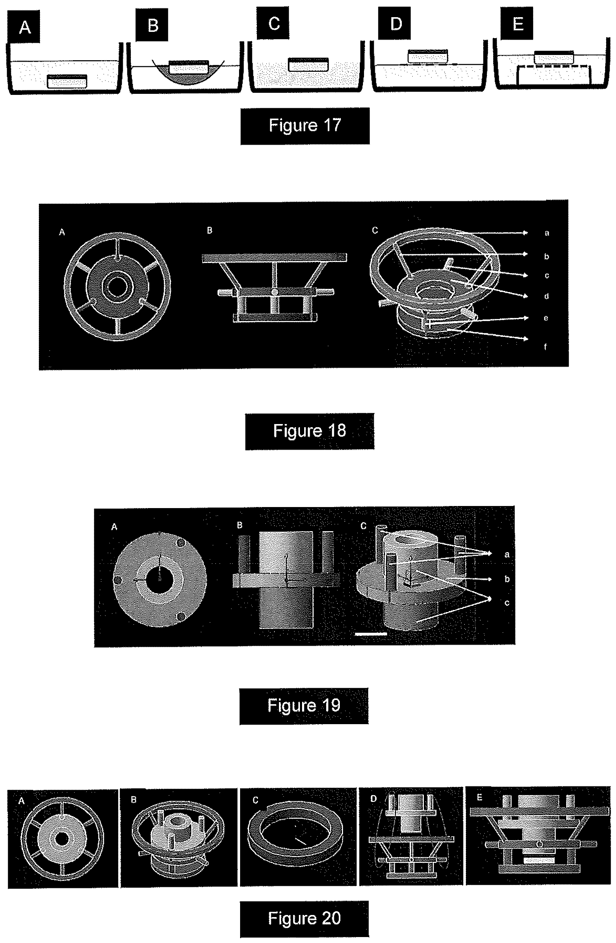

Figures of sketches 1 to 4 illustrate the lower part of the Epi-ExPer device. In particular,
   - Figure 1 is the top view of the lower part of the device according to the invention.
   - Figure 2 shows the side view of the lower part of the Epi-ExPer device.
   - Figure 3 shows the upper-side and figure 4 the bottom-side perspective view of the lower part of the Epi-ExPer device.
Figures 5 to 8 illustrate the upper part of the Epi-ExPer device according to the invention. In particular,

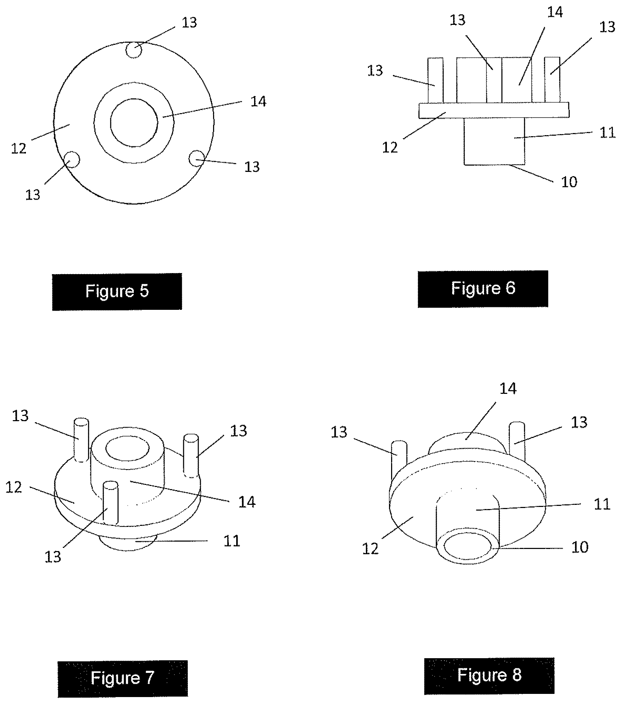

   - Figures 5 and 6 constitute the top view and side view of the upper part of the Epi-ExPer device, respectively.
   - Figures 7 and 8 show the top-side and the bottom-side perspective views of the upper part of the Epi-ExPer device, respectively.

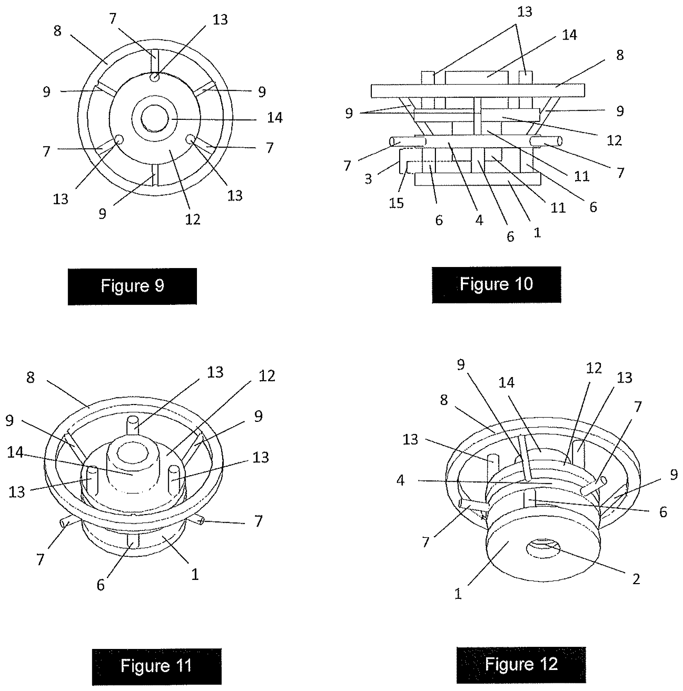

   - Figures 9 and 10 are the top and side view of the assembled Epi-ExPer device, respectively.
   - Figures 11 and 12 show the top-side and the bottom-side perspective views of the Epi-ExPer device, respectively.

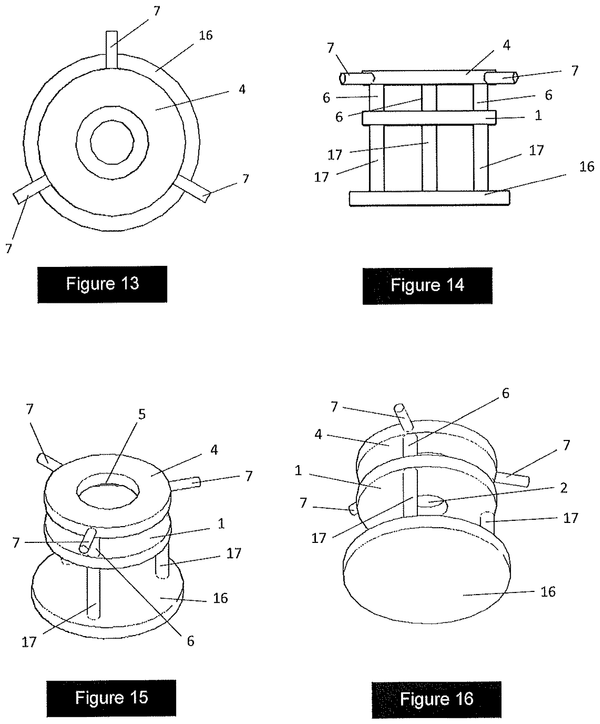

Figures 13 to 16 illustrate the lower part of the device according to an alternative embodiment of the device of the invention. In particular,
   - Figure 13 is the top view and figure 14 the side view of the lower part of the Epi-ExPer device variant.
   - Figure 15 shows the top-side and figure 16 the bottom-side perspective view of the lower part of the variant of the Epi-ExPer device.
Furthermore, the following figures are presented, in particular wherein
   - Figure 17 illustrates already established and known tissue/organ culture techniques, and is a schematic representation of existing organ/tissue culture models.

A shows a simple immersion of the epithelial tissue in liquid culture medium in contrast to other culture methods that facilitate culture conditions at an air-liquid interface (B-E).

B depicts the plasma clot method on a glass coverslip, C the agar gel method, D the raft method made of microscope lens paper/rayon acetate floating in culture medium, and finally E the metal grid method.
- Figure 18 illustrates the computer-aided design (CAD) drawing of the lower part of the Epi-ExPer device according to the invention. The top view (A), the side view (B) and the inclined top-side perspective view (C) are presented with the description of its individual parts, and in particular where (a) is the device mounting ring on the well rim, where (b) are the mounting ring supporting beams, where (c) are the cantilevers that retain the rubber bands, where (d) is the upper cylinder stabilization plane, where (e) are the supporting beams of the stabilization plane, and where (f) is the main plane for tissue placement.
- Figure 19 shows the CAD drawing of the upper part of the Epi-ExPer device. The top view (A), the side view (B) and the inclined top-side perspective view (C) are presented with the description of its individual parts, where (a) are the cantilevers for retaining the rubber bands, where (b) is the supporting cylinder plane of the cantilevers, and where (c) is the main cylinder/well.
- Figure 20 shows additional components and the assembly of the Epi-ExPer device. This is the CAD drawing of the assembled parts of the Epi-ExPer device. The top view (A) and angled view (B) of the assembled upper and lower parts of the device are shown, where the design of an elastic ring (C) that fits on the lower lip of the cylinder of the upper part of the device is shown, the upper and lower parts of the device in relative position to each other (D). On the lower lip of the cylinder of the upper part, the elastic 3D printed ring aims to prevent leakage from the upper part of the device. The dashed lines indicate the direction of insertion of the upper part and the solid lines indicate the position of the orthodontic rubber bands. The assembled Epi-ExPer device after the insertion of the epithelial tissue piece is also shown in a lateral view (E).

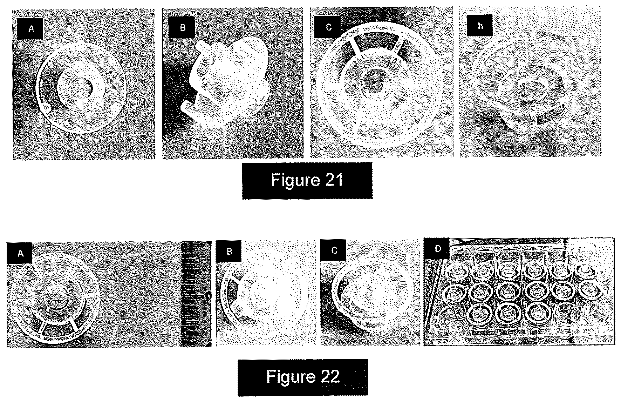

- Figure 21 shows the manufactured Epi-ExPer device, showing the upper (A and B) and lower (C and D) parts of the Epi-ExPer device. The upper (A and C) and angled (B and D) views are also shown.
- Figure 22 shows the assembly of the Epi-ExPer device, showing the lower part of the device after the placement of an epithelial tissue piece (A), the assembled device after the placement of a tissue piece and the attachment of the upper part to the tissue with the help of rubber bands in a top view (B) and at an angle (C), and finally fully assembled Epi-ExPer devices within a 24-well culture plate, the wells of which have been filled with liquid culture medium (D).

### Description of the Invention

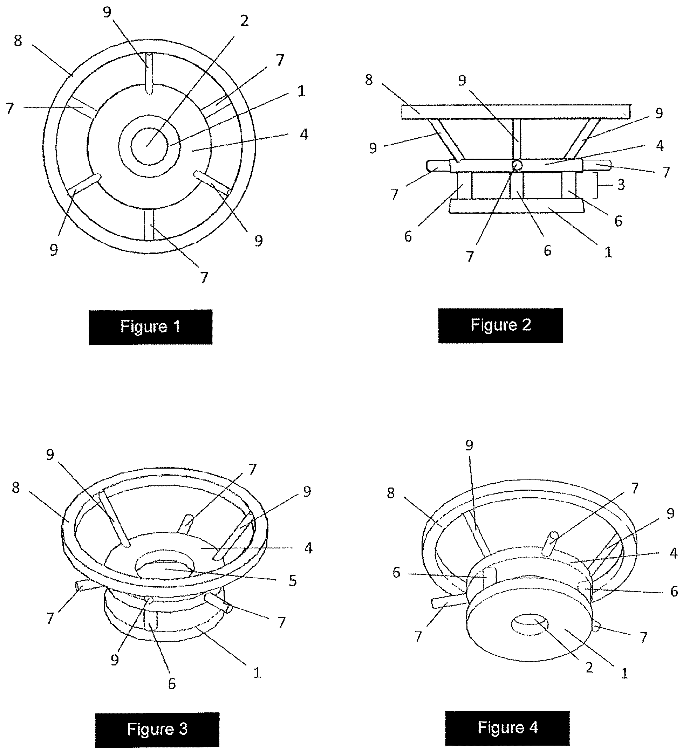

The device according to the invention consists of an upper part as shown in Figs. 5-8 and a lower part as shown in Figs. 1-4, which are assembled with the epithelial tissue piece and with each other, as shown in Figs. 9-11. According to a main embodiment of the invention, the tissue is placed inside the lower part, thus stabilizing both its position at the air-liquid interface condition and the orientation of the piece within a culture well during its cultivation period. At the same time, this lower part elevates the tissue within the culture well, so that the diffusing substance is collected in the underlying area, thus facilitating sampling and calculation of diffusion/permeability parameters. The lower part according to the main embodiment of the invention consists of a cylindrical plane 1, having in particular an outer diameter: 10 mm, inner diameter: 3 mm and height: 1 mm, and which constitutes the support plane of the tissue 1 and has a hole 2 in the center of diameter 3 mm, from which the tissue is supplied with nutrients, as it comes into contact with the underlying nutrient medium through the hole. The diameter of the hole 2 allows the smallest possible biopsy size of the tissue. Above the tissue support plane 1 there is an empty space 3 of 2 mm height for the placement of the tissue, while above this, a second cylindrical plane 4 of the same dimensions as those of the lower plane 1 is provided for receiving and stabilizing the upper part of the device, with the difference that the hole in the center of the cylinder 5 is 5 mm in diameter, so as to accommodate the lower cylinder 11 of the upper part, which has an external diameter of 4 mm. The two cylindrical planes are connected to each other by three beams 6, in particular of 1 mm diameter and 2 mm height. Three cantilevers 7 are provided on the periphery of the cylindrical stabilization and assembly plane, specifically with a diameter of 1 mm and a length of 3 mm for holding thin rubber bands that serve to hold the upper and lower parts together and in contact with the tissue. For tissue permeability control experiments, it is desirable to stir the fluid that has permeated the tissue, so that the underlying fluid volume is fully mixed and homogenized. For this reason, according to a preferred embodiment of the device according to the invention, an additional thin ring 8 is provided in the lower part of the device, which constitutes the plane that will fix the device and will be in direct contact with the rim of the well of commercially available 24-well culture plates. This ring 8, which has an internal diameter of 15 mm, sits on the rim of the well and the rest of the device hangs inside the well, like typical cell culture inserts. To hold this ring, three supporting beams 9, 1 mm thick, were placed diagonally, starting from the outer periphery of the stabilization plane 12 of the upper part and ending at the fixing ring 8. Thus, the design of the Epi-ExPer device allows the placement of a small magnet to stir the liquid that penetrates the barrier below the culture system and representative sampling for permeability measurement.

The upper part of the device holds the tissue piece on the base and has a well 11, which serves the targeted exposure of the exogenous factors under study on the outer surface of the epithelium. While the specimen is in culture, this well remains empty and therefore the epithelium is in contact with air (air-liquid interface potential), while when the specimen is exposed to the factors under study, the well is filled with liquid. Finally, this well serves to place the selected substance that will penetrate the tissue and based on which the epithelial diffusion/permeability coefficients will be calculated. In addition, a rubber ring is located on the lower lip 10 of the upper chamber of the device to prevent the possibility of leakage between the periphery of the upper cylinder and the tissue.

According to a main embodiment of the device according to the invention, the well of the upper part of the device consists downwardly of a tissue holding cylinder 11, in particular with an outer diameter of 4 mm, an inner diameter of 3 mm and a height of 2 mm, which fits on the periphery of the upper surface of the tissue piece and holds it in contact with the lower part of the device. On this was placed a plane 12, in particular with a diameter: 10 m, height: 1 mm, for the support of three cantilevers 13 (diameter: 1 mm, height: 3 mm) which hold the upper edge of the rubber bands. Finally, the tissue holding cylinder 11 expands upwards and thus creates a well for receiving the chemical exogenous exposure agents under study 14, specifically with an inner diameter: 3 mm, an outer diameter: 4 mm and a height: 3 mm. The well can remain empty during tissue culture steps, allowing tissue to be cultured at an air-liquid interface.

To ensure that liquid agent does not leak from the upper part of the device peripherally of the tissue, according to a further embodiment of the device according to the invention, there is additionally a thin elastic ring, which is welded to the lower edge 10 of the retaining cylinder 11 of the tissue of the upper part of the Epi-ExPer device. Specifically, these rings have a height of 0.5 mm, an inner diameter of 3 mm and an outer diameter of 3.5 mm.

In a main embodiment, the cultivation of epithelial tissue in a laboratory environment, whether for research or diagnostic purposes, is optimally done at an air-liquid interface, on culture plates/dishes in an incubator with conditions of 37 °C, 5% CO₂, and relative humidity. That means that the outer part of the epithelium is in contact with the air (dry environment), while it receives nutrients from the underlying liquid culture medium through the basement membrane of the epithelium. The epithelial tissue sample is taken from the patient using a circular-section scalpel (biopsy punch), transported to the laboratory and placed in a specific culture direction on a dish in a culture incubator. Exposure of epithelial tissue to exogenous chemicals should occur in contact only with the outer surface of the epithelium, thus simulating typical exposure conditions in the living organism. The effect of various exogenous factors as well as some pathological epithelial entities, e.g. diseases affecting epithelial tissue, can be evaluated through permeability testing.

During this test, substances of various molecular weights are exposed to the outer surface of the epithelial tissue and then the amount that penetrates the tissue and exits into the underlying space is measured and the various diffusion coefficients are calculated.

The respective epithelial tissue piece is placed on the placement plane 1 of the lower part (Fig. 1-4) of the device and then the upper part of the device is placed on the upper outer surface of the epithelium (Fig. 5-8), which is attached to the lower part of the device with the help of thin commercially available rubber bands. After assembling the tissue piece 15 with the Epi-ExPer device according to the invention, the system is placed in a 24-well plate, in a culture incubator (37°C, 5% CO₂, relative humidity) for the culture period. Each well under the Epi-ExPer device is filled with 2.4 mL of liquid nutrient medium. With this setup, the tissue piece receives nutrients only from its basal underlying membrane, while the upper outer surface of the epithelium remains dry in contact with the air. Thus, tissue culture is achieved at an air-liquid interface. In case it is desired to expose the tissue to exogenous chemicals, then the well 11 of the upper part of the device can receive a quantity of the liquid substance under study with a volume of 25-30µL. Finally, if tissue permeability testing is desired, then the well 11 of the upper part (Fig. 5-8) of the device can be filled with a corresponding substance, whose diffusion through the tissue and permeability through the epithelial barrier is measured by taking a sample from the underlying liquid, thus studying the amount of substance that has permeated the tissue. Typically, testing the permeability of a substance through a tissue involves sampling at short time intervals over several hours (e.g. 10, 20, 30, 60, 90, 120, 180, 240 minutes). Measurements of the diffusing substance can be made in various ways, e.g. with a spectrophotometer of a fluorescent substance.

The amount of the diffusing substance at each time point is placed on a graph and from the slope of the straight line of the graph, the diffusion constants are calculated, e.g. the steady-state flux parameter (Jss) and the apparent permeability coefficient parameter (Papp).

The small dimensions of the device serve to obtain small biopsies from donors, but in case larger surface tissues are needed for experimental purposes, the device can be enlarged while leaving its proportions and design the same.

The material used to make the prototype is a biocompatible polymer resin material and the manufacturing method was stereolithography 3D printing, however the material and manufacturing method are not restricting the invention.

Specifically, during manufacturing of the prototype Epi-ExPer devices, the design was initially done on a notebook and then, the two-dimensional drawings were transferred to three-dimensional format using a CAD software. The CAD files were exported from the software in .stl format, to be subsequently imported into PreForm software (Formlabs, Somerville, Massachusetts, USA). The Epi-ExPer device was fabricated using the Form 3B+ stereolithography 3D printer (Formlabs, MA, USA), without limiting the fabrication method of the Epi-ExPer device.

Elastic rings were fabricated using Elastic 50A resin (Formlabs, MA, USA). The printing time for the elastic rings for 30 devices is approximately 30 minutes. After printing was completed, the parts were removed from the printer platform using a thin spatula and placed in the From Wash chamber (Formlabs, MA, USA) in isopropyl alcohol solution for 20 min. The resin selected for printing the upper and lower parts of the Epi-ExPer device was the biocompatible resin BioMed Clear (Formlabs, MA, USA). The printing time for the main parts for 10 devices (five upper and five lower parts) is approximately 40 minutes.

After washing, the sections were left on a bench until the remaining alcohol was evaporated and then placed in the Form Cure polymerization device (Formlabs, MA, USA). Polymerization lasted 60 min for the upper and lower parts of the device while for the elastic rings it lasted 20 min.

After complete polymerization, the elastic rings were assembled and glued onto the lower surface of the cylinders of the upper parts of the device. The rings were glued using BioMed Clear resin and a small brush. The sections were then placed back in the From Cure device for re-polymerization for 30 min. Finally, the sections were cleaned with water and a brush and sterilized with ultraviolet radiation for 30 min on the upper and 30 min on the lower sides, before assembly with the tissue and further use.

In a variation of the Epi-ExPer device, for the most facilitating *ex vivo* culture of oral mucosa tissue sections depending on the desired experimental conditions, a variation of the basic design of the Epi-ExPer system was designed. In this variant, the Epi-ExPer device has all of its aforementioned features, apart from the ring 8 and its support beams 9, through which the device is placed on the well rim of commercially available culture dishes. Instead of the ring 8 and the beams 9, in the Epi-ExPer variant, a cylindrical bearing plane 16 of the device is provided on the bottom of the cultivation dish. The cylindrical plane 16 preferably has a diameter of 12 mm and a thickness of 1 mm. The plane 16 supports and elevates the device within the culture well and is specifically connected to the cylindrical tissue placement plane 1 via three beams 17, which have a diameter of 1 mm and a height of 5 mm. The upper part of the Epi-ExPer device variant remains the same as the aforementioned upper part of the Epi-ExPer device, and is connected in the same way to the corresponding lower part.

### References (scientific articles and mentioned tools):

*1. Scientific book chapter describing (p. 7) ex vivo organ*/*tissue culture techniques.* Malik, P., Mukherjee, T.K. (2023). Organ, Histotypic and Organotypic Culture, and Tissue Engineering. In: Mukherjee, T.K., Malik, P., Mukhopadhyay, S. (eds) Practical Approach to Mammalian Cell and Organ Culture. Springer, Singapore. https://doi.org/10.1007/978-981-19-1731-8 14-1
*2.* Cloning cylinders and rings by Fischer Scientific website link. https://www.fishersci.com/us/en/browse/90184185/cloning-rings-and-cylinders?page=1
*3.* High-vacuum silicone grease by Sigma Aldrich website link. https://www.sigmaaldrich.com/GR/en/product/aldrich/z273554
*4.* Protocol for the use of cloning rings by the production company Corning. https://www.fishersci.co.uk/content/dam/fishersci/en_EU/suppliers/corning/cc_cloning_prot ocol 1 03 cls an 041w.pdf
*5. Scientific article presenting an alternative use of cloning rings, and suggesting the replacement of vacuum glue with agarose gel.* Mathupala, S., & Sloan, A. A. (2009). An agarose-based cloning-ring anchoring method for isolation of viable cell clones. BioTechniques, 46(4), 305-307. https://doi.org/10.2144/000113079
*6. Scientific article with the application of vertical Franz cells for tissue permeability testing.* Salamanca, C. H., Barrera-Ocampo, A., Lasso, J. C., Camacho, N., & Yarce, C. J. (2018). Franz Diffusion Cell Approach for Pre-Formulation Characterisation of Ketoprofen SemiSolid Dosage Forms. Pharmaceutics, 10(3), 148. https://doi.org/10.3390/pharmaceutics10030148
*7.* Franz vertical diffusion cell by PermeGear website link. https://permegear.com/franz-cells/
*8. Scientific article presenting a variation of the vertical Franz cell, with the application of a microflow channel.* Pulsoni, I., Lubda, M., Aiello, M., Fedi, A., Marzagalli, M., von Hagen, J., & Scaglione, S. (2022). Comparison Between Franz Diffusion Cell and a novel Micro-physiological System for In Vitro Penetration Assay Using Different Skin Models. SLAS technology, 27(3), 161-171. https://doi.org/10.1016/j.slast.2021.12.006
*9. Scientific article presenting a 3D printed vertical Franz cell.* Sil, B. C., Alvarez, M. P., Zhang, Y., Kung, C. P., Hossain, M., Iliopoulos, F., Luo, L., Crowther, J. M., Moore, D. J., Hadgraft, J., Lane, M. E., & Hilton, S. T. (2018). 3D-printed Franz type diffusion cells. International journal of cosmetic science, 40(6), 604-609. https://doi.org/10.111/ics.12504
*10. Scientific article comparing a vertical Franz cell with a horizontal diffusion cell.* Bartos, C., Szabó-Révész, P., Horváth, T., Varga, P., & Ambrus, R. (2021). Comparison of Modern In Vitro Permeability Methods with the Aim of Investigation Nasal Dosage Forms. Pharmaceutics, 13(6), 846. https://doi.org/10.3390/pharmaceutics13060846
*11. Scientific article presenting a variation of the vertical Franz cell.* Sebe, I., Zsidai, L., & Zelkó, R. (2022). Novel modified vertical diffusion cell for testing of in vitro drug release (IVRT) of topical patches. HardwareX, 11, e00293. https://doi.org/10.1016/j.ohx.2022.e00293
*12. Editorial for the European 3R Directive on the use of laboratory animals.* Azkona G. (2023). Implementing the 3Rs in Laboratory Animal Research-From Theory to Practice. Animals : an open access journal from MDPI, 13(19), 3063. https://doi.org/10.3390/ani13193063

## Claims

1. Device for the controlled placement, cultivation, exposure and permeability assessment of *ex vivo* epithelial tissues, **characterised in that** it comprises an upper part and a lower part wherein on the one hand the upper part includes a retaining cylinder (11) which fits on the periphery of the upper surface of the epithelial tissue piece and holds it in contact with the lower part, here in particular on this upper part a flat cylinder (12) is placed for the support of three cantilevers (13) that hold the upper edge of the rubber bands, and where further the above tissue holding cylinder (11) extends upwards in such a way as to create a well (14), which either remains empty in the case of tissue culture in contact with air, or receives the chemical exogenous exposure factors under study, and on the other hand, the lower part includes a cylinder (1) which constitutes the support plane of the tissue and which has a hole in its center from which the tissue is supplied with nutrients, as it comes into contact with the underlying nutrient medium through it, where in particular a hole (3) is provided above it (1), and further above it (3) a second cylindrical plane (4) is provided with a bearing hole (5) for receiving and stabilizing the above upper part of the device, where on the cylindrical plane (4) three cantilevers (7) are specifically provided for holding rubber bands.

2. Device according to claim 1, **characterised in that** in the upper part the holding cylinder (11) has an outer diameter of particularly 4 mm, an inner diameter of 3 mm and a height of 2 mm, the cylindrical plane (12) has an outer diameter of 10 mm, a height of 1 mm, and bears in its center a hole of diameter 3 mm, which plane (12) serves to support the above cantilevers (13) which preferably have a diameter of 1 mm and a height of 3 mm, and where the receiving well (14) has an inner diameter of 3 mm, an outer diameter of 4 mm and a height of 3 mm.

3. Device according to any one of the preceding claims, **characterised in that** in the lower part the cylindrical supporting plane (1) has an outer diameter particularly of 10 mm, an inner diameter of 3 mm and a height of 1 mm, where in particular the hole of (2) is provided in the center preferably with a diameter of 3 mm, the second cylindrical plane (4) has an external diameter of 10 mm, while the hole of the cylinder (5) has a diameter of 5 mm.

4. Device according to any one of the preceding claims, **characterised in that** an additional thin ring is provided in the above upper part which is welded to the lower edge (10) of the above tissue holding cylinder (11), and fits to the periphery of the upper surface of the tissue piece.

5. Device according to any one of the preceding claims, **characterised in that** an additional thin ring (8) is provided in the lower part, which is connected to the cylindrical plane (4) by means of three beams (9) of preferably 0,5 mm diameter, and which ring (8) is intended to fix the device on the edge of a 24-well culture plate.

6. Device according to any one of the preceding claims, **characterised in that** the above cylinders (1) and (4) of the lower part are connected to each other by three beams (6), particularly with a diameter of 1 mm and a height of 2 mm.

7. Device according to any one of the preceding claims, **characterised in that** the above elastic ring that is welded to the rim (10) of the cylinder (11) of the upper part has a height of 0,5 mm, an internal diameter of 3 mm and an external diameter of 3.5 mm.

8. Device, particularly according to any one of the preceding claims, consisting of an upper and a lower part, **characterised by** its multiple role for controlled placement, culture, exposure and permeability assessment of *ex vivo* epithelial tissues, without the need to change the device configuration between roles.

9. Device according to claim 1, or according to any one of the claims 2 to 8, **characterised by** the placing, cultivating, exposing, and assessing the permeability of small epithelial tissue pieces, with a diameter even smaller than 5 mm, particularly as the hole diameter (2) of the plane (1) on which the tissue is held is 3 mm.

10. Device according to claim 1, or according to any one of the claims 2 to 9, **characterised by** culturing tissue sections in contact with a liquid-air interface, i.e. the upper part of the tissue can be cultured in contact with air through the well (14) provided in the upper part of the device, if desired, while the lower part of the tissue piece comes into contact with the liquid nutrient medium through the hole (2) of the plane (1) of the lower part of the device on which the tissue sits.

11. Device according to claim 1, or according to any one of the claims 2 to 10, **characterised by** a spatially controlled exposure of the outer surface of the epithelial tissue to exogenous factors, through the well (14) provided in the upper part of the device.

12. Device according to claim 1, or according to any one of the claims 2 to 11, **characterised by** a repeated use, as the upper and lower parts of the device can be disassembled and reassembled with a new tissue piece using thin rubber bands held on cantilevers in the upper (13) and lower parts (7) of the device.

13. Device according to claim 1, or according to any one of the claims 2 to 12, **characterised in that** an additional cylindrical solid plane (16), particularly 12 mm in diameter and 1 mm thick, is provided at the bottom of the device for its seating at the bottom of a well of a commercially available culture plate, wherein the device is placed within the culture well, rather than suspended therein via the ring (8) by which the device sits on the rim of commercially available 24-well culture plates, particularly wherein the main mounting plane (1) is fixed above the cylindrical bearing plane (16) with three beams (17), more particularly each of which has a diameter of 1 mm and a height of 5 mm.
